# EUROPÄISCHE PATENTSCHRIFT

(11) **EP 0 535 518 B1**
(45) Veröffentlichungstag und Bekanntmachung des Hinweises auf die Patenterteilung: **10.04.1996**
(21) Anmeldenummer: 92116238.4
(22) Anmeldetag: 23.09.1992
(51) Int. Cl.: C07D 211/26, C07D 213/38

(54) **Verfahren zur Herstellung von 2-Aminomethylpiperidin**
Process for the preparation of 2-piperidinemethanamine
Procédé pour la préparation de piperidine-2-méthanamine

(30) Priorität: 02.10.1991 DE 4132808
(43) Veröffentlichungstag der Anmeldung: 07.04.1993
(73) Patentinhaber: HOECHST AKTIENGESELLSCHAFT, D-65926 Frankfurt am Main (DE)
(72) Erfinder: Kampmann, Detlef, Dr., Dipl.-Chem., W-4630 Bochum (DE); Deckers, Gregor, Dr., Dipl.-Chem., W-4232 Xanten (DE); Kniep, Claus, W-4200 Oberhausen (DE)

(56) Entgegenhaltungen:
- FR-A- 1 530 809
- US-A- 3 246 000
- CHEMICAL ABSTRACTS, vol. 106, 1987, Columbus, Ohio, US; abstract no. 176178k, NAHATA,TOSHINARI ET AL. '( Aminomethyl)pyridines'
- CHEMICAL ABSTRACTS, vol. 106, 1987, Columbus, Ohio, US; abstract no. 176177j, IGUCHI, YOSHIO ET AL. '2- Or 3-(Aminomethyl)piperidine.'

## Beschreibung

Die vorliegende Erfindung betrifft ein Verfahren zur Herstellung von 2-Aminomethylpiperidin durch katalytische Hydrierung von 2-Cyanopyridin, die der nachstehend aufgeführten Reaktionsgleichung folgt:

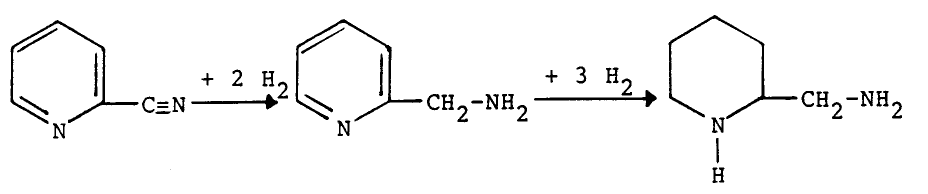

Die Umsetzung verläuft in Anwesenheit eines geeigneten Katalysators unter Druck und bei erhöhter Temperatur, wobei in einem ersten Schritt zunächst 2-Aminomethylpyridin als Zwischenprodukt gebildet wird. In einem nachfolgenden zweiten Hydrierschritt entsteht aus dem 2-Aminomethylpyridin das gewünschte 2-Aminomethylpiperidin.

Die EP 0 189 678 betrifft die elektrochemische Reduktion von 2-Cyanopyridin zu 2-Aminomethylpyridin; die Hydrierung von 2-Cyanopyridin mittels Palladium-AktivkohleKatalysatoren führt, wie aus US 4 153 605 und US 4 080 338 hervorgeht, überwiegend zu α-Bispicolylamin. 2-Aminomethylpiperidin wird hingegen nur in untergeordneten Mengen gebildet.

Die Umsetzung von 2-Aminomethylpyridin zu 2-Aminomethylpiperidin mittels PtO₂-Katalysatoren in essigsaurer, wäßriger Lösung ist in US 3 772 230 und US 3 631 046 beschrieben. Die erforderliche abschließende Aufarbeitung - Freisetzen des Amins mit Kaliumhydroxid, Extraktion mit Diethylether, Trocknung der Etherphase mit Hilfe von Natriumsulfat und fraktionierte Destillation - ist allerdings sehr aufwendig.

Ein weiteres Verfahren zur Herstellung von 2-Aminomethylpiperidin ist Inhalt der JP 61/251 663 (86/251 663) und JP 61/251 659 (86/251 659).Gemäß JP 61/251 663 wird eine Lösung von 2-Cyanopyridin in Benzol unter Zusatz von Ammoniak mittels Raney-Nickel zu 2-Aminomethylpyridin hydriert. Anschließend wird entsprechend JP 61/251 659 in einem separaten Schritt das 2-Aminomethylpyridin in wäßriger Phase in Gegenwart von Rhodium-Aktivkohle als Katalysator mit Wasserstoff zu 2-Aminomethylpiperidin umgesetzt. Wegen der Verwendung zweier unterschiedlicher Katalysatoren und der Notwendigkeit in zwei verschiedenen Medien - benzolische Lösung einerseits, wäßrige Phase andererseits - zu arbeiten, ist das Verfahren recht umständlich und wenig praxisfreundlich. Zudem ist die abschließende Abtrennung des 2-Aminomethylpiperidins aus der wäßrigen Lösung mit erheblichem Aufwand verbunden.

Es besteht daher ein Bedarf an einem Verfahren, das die Nachteile der vorstehend genannten Verfahren vermeidet und einen Weg eröffnet, unter Einsatz ein und desselben Katalysators und ohne Wechseln des Lösungsmittels 2-Cyanopyridin zu 2-Aminomethylpiperidin zu hydrieren. Das Verfahren soll sich eines in kommerziellen Mengen zur Verfügung stehenden Katalysators bedienen und sich mit vertretbarem Aufwand auch in technischem Umfang realisieren lassen. Darüber hinaus soll gewährleistet sein, daß die Umsetzung nicht zur Bildung größerer Mengen Nebenprodukte führt, sondern das gewünschte Wertprodukt, nämlich 2-Aminomethylpiperidin, in guter Ausbeute liefert.

Diese Aufgabe wird gelöst durch ein Verfahren zur Herstellung von 2-Aminomethylpiperidin durch katalytische Hydrierung von 2-Cyanopyridin. Es ist dadurch gekennzeichnet, daß man 2-Cyanopyridin mit Wasserstoff in Gegenwart eines Cobalt enthaltenden Katalysators bei 120 bis 230°C und 10 bis 45 MPa umsetzt.

Als Cobalt enthaltender Katalysator kommen die üblichen, im Handel befindlichen Cobalt-Katalsatoren, die in technischen Mengen lieferbar sind in Betracht. Bei Auswahl geeigneter Cobalt-Katalysatoren sollte darauf geachtet werden, daß der Katalysator Cobalt in ausreichender Menge enthält. Im allgemeinen sollte der Cobalt-Anteil 25 bis 85, insbesondere 30 bis 75, bevorzugt 40 bis 60 Masse-% Cobalt, bezogen auf die gesamte Katalysatormasse, betragen.

Neben Cobalt können die Katalysatoren noch Aktivatoren und Promotoren enthalten. Als Aktivator eignen sich Zr, Mn, Cr, V, insbesondere Zr, Mn, Cr bevorzugt Zr und Mn. Brauchbar als Promotor sind Alkali- und/oder Erdalkalimetalle, insbesondere Li, Na, K und/oder Mg, Ca, Ba, bevorzugt Na, K und/oder Mg, Ca. Der Katalysator weist sowohl die Aktivatoren als auch die Promotoren in den üblichen Mengen auf. Ihr Anteil beträgt in Summe 2,5 bis 40, insbesondere 5 bis 35, bevorzugt 10 bis 30 Masse-%, bezogen auf die gesamte Katalysatormasse.

Besonders vorteilhaft ist der Gebrauch von Trägerkatalysatoren als Cobalt enthaltender Katalysator. Derartige Trägerkatalysatoren lassen sich nach bekannten Methoden, beispielsweise durch Fällung und durch Tränkung herstellen.

Als Trägermaterial können Al₂O₃, Aktivkohle, ein Silikat, Kieselgel oder Kieselgur, insbesondere ein Silikat, wie ein Ca-, Mg- oder Al-silikat, Kieselgel oder Kieselgur, bevorzugt Kieselgel oder Kieseigur, besonders bevorzugt Kieselgur verwendet werden. Der Cobalt enthaltende Katalysator besteht zu 15 bis 65, insbesondere 20 bis 60, bevorzugt 20 bis 50 Masse-% aus Trägermaterial, bezogen auf gesamte Katalysatormasse.

Der Cobalt enthaltende Katalysator kann sowohl in stabilisierter Form als auch als pyrophores Produkt eingesetzt werden. Wegen der einfacheren Handhabung wird häufig einem stabilisierten, das heißt gegenüber der Einwirkung von Luft inert gemachten Katalysator der Vorzug gegeben. Es hat sich jedoch herausgestellt, daß pyrophore Cobalt enthaltende Katalysatoren meist bessere Resultate als entsprechende stabilisierte Katalysatoren liefern. Aus diesem Grund sind pyrophore Cobalt-Katalysatoren besonders zu empfehlen.

Ein weiterer Vorzug des erfindungsgemäßen Verfahrens besteht darin, daß es sich besonders flexibel gestalten läßt. Es beschränkt sich nämlich nicht ausschließlich auf einen zweistufigen Prozess, wobei zunächst 2-Cyanopyridin zu 2-Aminomethylpyridin und dieses anschließend zu 2-Aminomethylpiperidin hydriert wird, sondern es läßt sich besonders einfach als eine einstufige Umsetzung anwenden. Diese einstufige Verfahrensvariante kommt den Anforderungen, die an einen technischen Prozeß gestellt werden, besonders entgegen, da sie sich - verglichen mit einem zweistufigen Verfahren - wesentlich einfacher durchführen läßt und zudem auch den apparativen Aufwand nicht unerheblich reduziert.

Das erfindungsgemäße Verfahren kann unabhängig von einer zweistufigen oder einstufigen Ausführungsform sowohl diskontinuierlich, beispielsweise in einem druckfesten Rührbehälter oder Autoklaven, oder kontinuierlich ausgeübt werden. Es ist besonders für einen kontinuierlichen Prozeß geeignet.

Bei einem kontinuierlichen Prozeß verwendet man üblicherweise druckfeste Rohrreaktoren, in denen der Cobalt enthaltende Katalysator in stückiger Form als Festbett angeordnet vorliegt. Die Ausgangsstoffe, 2-Cyanopyridin und Wasserstoff, werden dem Rohrreaktor entweder am Kopf oder am Boden zugeführt. Je nach Art der Zugabe spricht man von einer Riesel- oder Sumpffahrweise. Das Reaktionsgemisch verläßt den Rohrreaktor entsprechend der Zugabe der Ausgangsstoffe entweder am Boden oder am Kopf. Die Umsetzung kann sowohl im geraden Durchgang als auch unter Zuhilfenahme eines Reaktionsproduktkreislaufes durchgeführt werden.

Nach einer besonderen Ausführungsform werden die Einsatzstoffe dem Rohrreaktor am Boden zugeleitet und die Umsetzung erfolgt im geraden Durchgang, d. h. ohne einen Teil des Reaktionsproduktes im Kreis zu führen. Das Reaktionsgemisch verläßt den Rohrreaktor am Kopf.

Die Reaktionsbedingungen, insbesondere Druck und Temperatur, sind zum einen davon abhängig, ob das Verfahren zweistufig oder einstufig abläuft, zum anderen werden sie auch in gewissem Umfange von der Art des Cobalt enthaltenden Katalysators beeinflußt und müssen entsprechend aufeinander abgestimmt werden.

Wählt man ein zweistufiges Verfahren, so führt man die Umsetzung in der ersten Stufe bei 120 bis 160, insbesondere 130 bis 150, bevorzugt 135 bis 145°C und 10 bis 20, insbesondere 10 bis 15 MPa und in der zweiten Stufe bei 160 bis 230, insbesondere 170 bis 200, bevorzugt 175 bis 190°C und 20 bis 45, insbesondere 25 bis 40, bevorzugt 25 bis 35 MPa durch.

Bei Anwendung eines einstufigen Verfahrens führt man die Umsetzung in einer einzigen Stufe bei 160 bis 230, insbesondere 170 bis 200, bevorzugt 175 bis 195°C und 20 bis 45, insbesondere 25 bis 40, bevorzugt 25 bis 35 MPa durch.

Um die Hydrierung günstig zu beeinflussen, setzt man Wasserstoff, bezogen auf den stöchiometrischen Bedarf, im Überschuß ein. Unverbrauchter Wasserstoff wird aus dem den Reaktor verlassenden Reaktionsprodukt, beispielsweise mittels eines Hochdruckgasabscheiders und eines nachgeschalteten Niederdruckgasabscheiders, abgetrennt und gegebenenfalls nach Kompression in die Reaktion wiedereingesetzt.

Zur Unterdrückung von Reaktionen, die zu einer Abspaltung von Ammoniak führen, kann man das Verfahren unter Zusatz von Ammoniak, das erforderlichenfalls dem Wasserstoffgas zugesetzt wird, ablaufen lassen. Es hat sich jedoch gezeigt, daß in den meisten Fällen auf eine Zugabe von Ammoniak verzichtet werden kann, ohne eine Verschlechterung der Umsetzung in Kauf nehmen zu müssen.

Bei 2-Cyanopyridin handelt es sich um einen Feststoff mit einem Schmelzpunkt von etwa 28 bis 30°C. Um 2-Cyanopyridin leichter zu handhaben, empfiehlt es sich, ein Lösungsmittel zu verwenden.

Je Masse-Teil 2-Cyanopyridin setzt man 0,5 bis 5, insbesondere 1,0 bis 3,0 bevorzugt 1,5 bis 2,5 Masse-Teile Lösungsmittel ein. Das Lösungsmittel soll im wesentlichen gegenüber der Umsetzung inert sein, d. h. nicht oder nur in geringem Umfange einer Hydrierung unterliegen und keine Reaktion mit dem Einsatzstoff und dem sich bildenden Reaktionsgemisch eingehen.

Geeignete Solventien sind Tetrahydrofuran, Dioxan, Toluol, Xylol oder Cumol, insbesondere Tetrahydrofuran, Dioxan oder Toluol, bevorzugt Toluol. Es können auch Gemische vorstehend genannter Solventien Anwendung finden.

Zur Reindarstellung von 2-Aminomethylpiperidin wird das Reaktionsgemisch nach Abtrennung gasförmiger Bestandteile, beispielsweise Wasserstoff und/oder Ammoniak, einer fraktionierten Destillation unterworfen.

2-Aminomethylpiperidin findet als Zwischenprodukt für die Herstellung von Arzneimitteln Verwendung.

Die nachstehend beschriebenen Beispiele belegen die vorliegende Erfindung.

### Experimenteller Teil

### Vergleichsversuche

### Vergleichsversuch 1 a) Herstellung von 2-Aminomethylpyridin

In einem mit einem Hubrührer bestückten Autoklav (Volumen 1 l) werden 52 g Cyanopyridin, 260 g Toluol und 5 g eines Nickelkatalysators, der etwa 50 bis 53 Masse-% Ni und 25 bis 30 Masse-% Kieselgur als Träger enthält, vorgelegt.

Anschließend werden 65 g NH₃ (entsprechend 7,6 Mol NH₃ je Mol Cyanopyridin) zugesetzt und es wird Wasserstoff bis zum Erreichen eines Vordrucks von 3,0 MPa aufgepreßt.

Man heizt unter Rühren auf und hält den für die Durchführung der Reaktion erforderlichen Druck durch Zugabe von Wasserstoff konstant.

### Reaktionsbedingungen:

| | |
|---|---|
| Druck | 10 MPa |
| Temperatur | 120°C |
| Reaktionszeit | 3 h |

Nach Abschluß der Reaktion wird das Reaktionsgemisch von überschüssigem NH₃ und Wasserstoff befreit und vom Katalysator durch Filtration abgetrennt.

### Vergleichsversuch 1 b) Herstellung von 2-Aminomethylpiperidin

In einem mit einem Hubrührer bestückten Autoklav (Volumen 0,25 l) werden 116 g des aus Vergleichsversuch 1 a) resultierenden Reaktionsgemisches mit 2 g eines Rhodium-katalysators (4,83 Masse-% Rh auf Aktivkohle als Träger) vorgelegt.

Anschließend wird Wasserstoff bis zum Erreichen eines Vordrucks von 2,0 MPa aufgepreßt.

Man heizt unter Rühren auf und hält den für die Durchführung der Reaktion erforderlichen Druck durch Zugabe von Wasserstoff konstant.

### Reaktionsbedingungen:

| | |
|---|---|
| Druck | 4 MPa |
| Temperatur | 110°C |
| Reaktionszeit | 2 h |

### Vergleichsversuch 1 c) Herstellung von 2-Aminomethylpiperidin entsprechend US 3 772 230

In einem mit einem Hubrührer bestückten Autoklav (Volumen 0,25 l) werden 48,2 g des aus Vergleichsversuch 1 a) resultierenden Reaktionsgemisches, 86 g Essigsäure und 5 g eines Platinkatalysators, (etwa 5 Masse-% Pt auf Aktivkohle als Träger) vorgelegt.

Anschließend wird Wasserstoff bis zum Erreichen eines Vordrucks von 0,2 MPa aufgepreßt.

Man heizt unter Rühren auf und hält den für die Durchführung der Reaktion erforderlichen Druck durch Zugabe von Wasserstoff konstant.

### Reaktionsbedingungen:

| | |
|---|---|
| Druck | 0,35 MPa |
| Temperatur | 50°C |
| Reaktionszeit | 5 h |

### Vergleichsversuch 2 Herstellung von 2-Aminomethylpyridin

In einem mit einem Hubrührer bestückten Autoklav (Volumen 1 l) werden 208 g Cyanopyridin, 208 g Toluol und 20,8 g Raney-Ni vorgelegt.

Anschließend werden 340 g NH₃ (entsprechend 10 Mol NH₃ je Mol Cyanopyridin) zugesetzt und es wird Wasserstoff bis zum Erreichen eines Vordrucks von 3,0 MPa aufgepreßt.

Man heizt unter Rühren auf und hält den für die Durchführung der Reaktion erforderlichen Druck durch Zugabe von Wasserstoff konstant.

### Reaktionsbedingungen:

| | |
|---|---|
| Druck | 10 MPa |
| Temperatur | 90°C |
| Reaktionszeit | 4 h |

Nach Abschluß der Reaktion wird das Reaktionsgemisch von überschüssigem NH₃ und Wasserstoff befreit und vom Katalysator durch Filtration abgetrennt.

### Beispiele

### Beispiel 1 a) Herstellung von 2-Aminomethylpyridin

In einem mit einem Hubrührer bestückten Autoklav (Volumen 5 l) werden 416 g Cyanopyridin 832 g Toluol und 83,2 g eines pyrophoren Cobaltkatalysators, der etwa 44 bis 47 Masse-% Co und 25 bis 30 Masse-% Kieselgur als Träger enthält, vorgelegt. Auf einen Zusatz von NH₃ wird verzichtet.

Man preßt Wasserstoff auf, heizt unter Rühren auf und hält den für die Durchführung der Reaktion erforderlichen Druck durch Zugabe von Wasserstoff konstant.

### Reaktionsbedingungen:

| | |
|---|---|
| Druck | 10 MPa |
| Temperatur | 140°C |
| Reaktionszeit | 50 min |

Nach Abschluß der Reaktion wird das Reaktionsgemisch von überschüssigem Wasserstoff befreit und vom Katalysator durch Filtration abgetrennt.

### Beispiel 1 b) Herstellung von 2-Aminomethylpiperidin

In einem mit einem Hubrührer bestückten Autoklav (Volumen 0,25 l) werden 72 g des aus Beispiel 1 a) resultierenden Reaktionsgemisches und 5,2 g eines stabilisierten Cobaltkatalysators, der etwa 44 bis 47 Masse-% Co und 25 bis 30 Masse-% Kieselgur als Träger enthält vorgelegt. Auf einen Zusatz von NH₃ wird verzichtet.

Man preßt Wasserstoff auf, heizt unter Rühren auf und hält den für die Durchführung der Reaktion erforderlichen Druck durch Zugabe von Wasserstoff konstant.

### Reaktionsbedingungen:

| | |
|---|---|
| Druck | 25 MPa |
| Temperatur | 180°C |
| Reaktionsdauer | 8 h 40 min |

### Beispiel 2 Einstufige Herstellung von 2-Aminomethylpiperidin

In einem mit einem Hubrührer bestückten Autoklav (Volumen 0,25 l) werden 26 g Cyanopyridin, 52 g Toluol und 5,1 g des in Beispiel 1 a) genannten, pyrophoren Cobaltkatalysators, der etwa 44 bis 47 Masse-% Co und 25 bis 30 Masse-% Kieselgur als Träger enthält, vorgelegt. Auf einen Zusatz von NH₃ wird verzichtet.

Man preßt Wasserstoff auf, heizt unter Rühren auf und hält den für die Durchführung der Reaktion erforderlichen Druck durch Zugabe von Wasserstoff konstant.

### Reaktionsbedingungen:

| | |
|---|---|
| Druck | 25 MPa |
| Temperatur | 180°C |
| Reaktionszeit | 3,5 h |

### Beispiel 3 Einstufige Herstellung von 2-Aminomethylpiperidin

In einem mit einem Hubrührer bestückten Autoklav (Volumen 5 l) werden 624 g Cyanopyridin, 1248 g Toluol und 125,6 g des in Beispiel 1 a) genannten, pyrophoren Cobaltkatalysators, der etwa 44 bis 47 Masse-% Co und 25 bis 30 Masse-% Kieselgur als Träger enthält, vorgelegt. Auf einen Zusatz von NH₃ wird verzichtet.

Man preßt Wasserstoff auf, heizt unter Rühren auf und hält den für die Durchführung der Reaktion erforderlichen Druck durch Zugabe von Wasserstoff konstant.

### Reaktionsbedingungen:

| | |
|---|---|
| Druck | 25 MPa |
| Temperatur | 180°C |
| Reaktionszeit | 3 h 40 min |

Die Ergebnisse der Vergleichsversuche und der Beispiele sind in der nachfolgenden Tabelle zusammengestellt.

**Tabelle**

| Zusammensetzung¹⁾ | Vergleichsversuche | | | | Beispiele | | | |
|---|---|---|---|---|---|---|---|---|
| | 1a | 1b | 1c | 2 | 1a | 1b | 2 | 3 |
| Vorlauf | 2,2 | - | 0,4 | 0,5 | 4,6 | 32,2 | 30,9 | 26,8 |
| 2-Aminomethylpiperidin | - | 14,4 | 6,2 | <0,01 | - | 57,0 | 60,1 | 61,6 |
| Komponente | - | - | - | 0,3 | - | - | - | <0,1 |
| 2-Aminomethylpyridin | 76,8 | 40,5 | 51,5 | 62,0 | 88,4 | 1,5 | 0,3 | 0,6 |
| 2-Cyanopyridin | - | - | - | 23,1 | - | - | 0,2 | - |
| Höhersiedende Anteile | 21,0 | 45,1 | 41,9 | 14,1 | 7,0 | 9,3 | 8,5 | 10,9 |
| Umsatz²⁾ (%) | 100,0 | 100,0 | 100,0 | 76,9 | 100,0 | 100,0 | 99,8 | 100,0 |
| Selektivität^{*} (%) | 76,8 | 40,5 | 51,5 | 80,6 | 88,5 | 1,5 | 0,3 | 0,6 |
| Selektivität^{**} (%) | - | 14,4 | 6,2 | - | - | 57,0 | 60,3 | 61,6 |
| Ausbeute³⁾ (%) | 76,8 | - | - | 62,0 | 88,5 | 1,5 | 0,3 | 0,6 |
| Ausbeute⁴⁾ | - | 14,4 | 6,2 | - | - | 57,0 | 60,1 | 61,6 |

| | | | | | | | | |
|---|---|---|---|---|---|---|---|---|
| 1) gaschromatographische Analyse in Masse-% ohne Lösungsmittel und Ammoniak | | | | | | | | |
| 2) bezogen auf 2-Cyanopyridin | | | | | | | | |
| 3) 2-Aminomethylpyridin bezogen auf 2-Cyanopyridin | | | | | | | | |
| 4) 2-Aminomethylpiperidin bezogen auf 2-Cyanopyridin | | | | | | | | |
| * Bildung von 2-Aminomethylpyridin | | | | | | | | |
| ** Bildung von 2-Aminomethylpiperidin | | | | | | | | |

## Patentansprüche

1. Verfahren zur Herstellung von 2-Aminomethylpiperidin durch katalytische Hydrierung von 2-Cyanopyridin, dadurch gekennzeichnet, daß man 2-Cyanopyridin mit Wasserstoff in Gegenwart eines Cobalt enthaltenden Katalysators bei 120 bis 230°C und 10 bis 45 MPa umsetzt.

2. Verfahren nach Anspruch 1, dadurch gekennzeichnet, daß der Cobalt enthaltende Katalysator wenigstens 25 bis 85, insbesondere 30 bis 75, bevorzugt 40 bis 60 Masse-% Cobalt aufweist.

3. Verfahren nach Anspruch 1 oder 2, dadurch gekennzeichnet, daß der Cobalt enthaltende Katalysator ein Trägerkatalysator ist.

4. Verfahren nach einem oder mehreren der Ansprüche 1 bis 3, dadurch gekennzeichnet, daß der Cobalt enthaltende Katalysator Al₂O₃, Aktivkohle, ein Silikat, Kieselgel oder Kieselgur, insbesondere ein Silikat, Kieselgel oder Kieselgur, bevorzugt Kieselgur als Trägermaterial enthält.

5. Verfahren nach einem oder mehreren der Ansprüche 1 bis 4, dadurch gekennzeichnet, daß der Cobalt enthaltende Katalysator 5 bis 60, insbesondere 10 bis 50, bevorzugt 15 bis 50 Masse-% Trägermaterial aufweist.

6. Verfahren nach einem oder mehreren der Ansprüche 1 bis 5, dadurch gekennzeichnet, daß man die Umsetzung bei 120 bis 160, insbesondere 130 bis 150, bevorzugt 135 bis 145°C und 10 bis 20 , insbesondere 10 bis 15 MPa und anschließend bei 160 bis 230, insbesondere 170 bis 200, bevorzugt 175 bis 190°C und 20 bis 45, insbesondere 25 bis 40, bevorzugt 25 bis 35 MPa durchführt.

7. Verfahren nach einem oder mehreren der Ansprüche 1 bis 5, dadurch gekennzeichnet, daß man die Umsetzung in einer einzigen Stufe bei 160 bis 230, insbesondere 170 bis 200, bevorzugt 175 bis 190°C und 20 bis 45, insbesondere 25 bis 40, bevorzugt 25 bis 35 MPa durchführt.

8. Verfahren nach einem oder mehreren der Ansprüche 1 bis 7, dadurch gekennzeichnet, daß man die Umsetzung in Gegenwart eines gegenüber der Umsetzung im wesentlichen inerten Lösungsmittels oder Lösungsmittelgemisches durchführt.

9. Verfahren nach einem oder mehreren der Ansprüche 1 bis 8, dadurch gekennzeichnet, daß man als Lösungsmittel Tetrahydrofuran, Dioxan, Toluol, Xylol oder Cumol, insbesondere Tetrahydrofuran, Dioxan oder Toluol, bevorzugt Toluol einsetzt.

## Claims

1. A process for the preparation of 2-aminomethyl piperidine by the catalytic hydrogenation of 2-cyano pyridine, which comprises reacting 2-cyanopyridine with hydrogen in the presence of a cobalt-containing catalyst at 120 to 230°C and 10 to 45 MPa.

2. The process as claimed in claim 1, wherein the cobalt-containing catalyst contains at least 25 to 85, especially 30 to 75 and preferably 40 to 60% by weight of cobalt.

3. The process as claimed in claim 1 or 2, wherein the cobalt-containing catalyst is a supported catalyst.

4. The process as claimed in one or more of claims 1 to 3, wherein the cobalt-containing catalyst contains Al₂O₃, activated charcoal, a silicate, silica gel or kieselguhr, especially a silicate, silica gel or kieselguhr and preferably kieselguhr as the support.

5. The process as claimed in one or more of claims 1 to 4, wherein the cobalt-containing catalyst contains 5 to 60, especially 10 to 50 and preferably 15 to 50% by weight of support.

6. The process as claimed in one or more of claims 1 to 5, wherein the reaction is carried out at 120 to 160, especially 130 to 150 and preferably 135 to 145°C and at 10 to 20 and especially 10 to 15 MPa, and then at 160 to 230, especially 170 to 200 and preferably 175 to 190°C and at 20 to 45, especially 25 to 40 and preferably 25 to 35 MPa.

7. The process as claimed in one or more of claims 1 to 5, wherein the reaction is carried out in a single stage at 160 to 230, especially 170 to 200 and preferably 175 to 190°C and at 20 to 45, especially 25 to 40 and preferably 25 to 35 MPa.

8. The process as claimed in one or more of claims 1 to 7, wherein the reaction is carried out in the presence of a solvent or solvent mixture which is essentially inert towards the reaction.

9. The process as claimed in one or more of claims 1 to 8, wherein the solvent used is tetrahydrofuran, dioxane, toluene, xylene or cumene, especially tetrahydrofuran, dioxane or toluene and preferably toluene.

## Revendications

1. Procédé pour la fabrication de 2-aminométhylpipéridine par hydrogénation catalytique de la 2-cyanopyridine, caractérisé en ce que l'on fait réagir la 2-cyanopyridine avec l'hydrogène à 120-230°C et sous 10-45 MPa en présence d'un catalyseur au cobalt.

2. Procédé selon la revendication 1, caractérisé en ce que le catalyseur au cobalt contient au moins 25 à 85 % en masse, en particulier 30 à 75 % en masse, de préférence 40 à 60 % en masse de cobalt.

3. Procédé selon la revendication 1 ou 2, caractérisé en ce que le catalyseur au cobalt est un catalyseur sur support.

4. Procédé selon une ou plusieurs des revendications 1 à 3, caractérisé en ce que le catalyseur au cobalt contient comme matière de support Al₂O₃, du charbon actif, un silicate, du gel de silice ou de la terre d'infusoires, en particulier un silicate, du gel de silice ou de la terre d'infusoires, de préférence de la terre d'infusoires.

5. Procédé selon une ou plusieurs des revendications 1 à 4, caractérisé en ce que le catalyseur au cobalt contient 5 à 60 % en masse, en particulier 10 à 50 % en masse, de préférence 15 à 50 % en masse de matière de support.

6. Procédé selon une ou plusieurs des revendications 1 à 5, caractérisé en ce que l'on met en oeuvre la réaction à 120-160°C, en particulier 130-150°C, de préférence 135-145°C et sous 10-20 MPa, en particulier 10-15 MPa et ensuite à 160-230°C, en particulier 170-200°C, de préférence 175-190°C et sous 20-45 MPa, en particulier 25-40 MPa, de préférence 25 à 35 MPa.

7. Procédé selon une ou plusieurs des revendications 1 à 5, caractérisé en ce que l'on met en oeuvre la réaction en une seule étape à 160-230°C, en particulier 170-200°C, de préférence 175-190°C et sous 20-45 MPa, en particulier 25-40 MPa, de préférence 25-35 MPa.

8. Procédé selon une ou plusieurs des revendications 1 à 7, caractérisé en ce que l'on met en oeuvre la réaction en présence d'un solvant ou mélange solvant essentiellement inerte vis-à-vis de la réaction.

9. Procédé selon une ou plusieurs des revendications 1 à 8, caractérisé en ce que l'on utilise comme solvant le tétrahydrofurane, le dioxane, le toluène, le xylène ou le cumène, en particulier le tétrahydrofurane, le dioxane ou le toluène, de préférence le toluène.
